# EUROPEAN PATENT APPLICATION

(11) **EP 4 374 785 A1**
(43) Date of publication of application: **29.05.2024**
(21) Application number: 22209259.5
(22) Date of filing: 24.11.2022
(51) Int. Cl.: A61B 5/11

(54) **TRACKING PHYSIOLOGICAL WELLBEING**

(71) Applicant: Koninklijke Philips N.V., 5656 AG Eindhoven (NL)
(72) Inventor: KUHLMANN, Daan, Eindhoven (NL); GELISSEN, Jozef Hubertus, Eindhoven (NL)
(74) Representative: Philips Intellectual Property & Standards

(57) **Abstract**

A method and system for tracking physiological wellbeing of a user during an activity. Information relating to micro-movements, biological parameters and activity performance of the user during the activity is received and processed to determine a physiological wellbeing value.

## Description

### FIELD OF THE INVENTION

This invention relates to the field of tracking physiological wellbeing of a user, and in particular to the field of tracking physiological wellbeing of a user during an activity.

### BACKGROUND OF THE INVENTION

Users, while engaged in an activity, often forget to eat and drink enough. Example activities include working at a computer, gaming, or prolonged activity on social media. It is known that not eating enough can lead to a reduction in physiological wellbeing, such as malnutrition and this can lead to low blood sugar and vitamin deficiency. As a consequence to this, the body can become shaky. Similarly, dehydration can cause uncontrolled shaking, especially evident in the hands of a user. It may therefore be possible to track dehydration and malnutrition of a user in order to provide feedback to the user.

However, shaking may also occur due to factors other than malnutrition or dehydration, such as stress, for instance when a user is playing a game, making a direct correlation between shaking and dehydration or malnutrition difficult.

Hence, there is a need for a solution to reliably track the physiological wellbeing of a user during an activity.

### SUMMARY OF THE INVENTION

The invention is defined by the claims.

According to examples in accordance with an aspect of the invention, there is provided a method for tracking physiological wellbeing of a user during an activity, the method comprising: receiving shakiness information relating to micro-movements of the user from at least one movement sensor; receiving biological information relating to biological parameters of the user from at least one biological sensor; receiving performance information relating to a level of performance of the user in performing the activity from a system which the user operates to perform the activity; and processing the shakiness information, biological information, and performance information to determine a physiological wellbeing value.

Tracking physiological wellbeing is of particular interest for monitoring subjects who may forget to drink or eat during the day, in particular when they are engrossed in a particular activity which lasts a long time. Examples include working at a computer, gaming, or prolonged activity on social media. It is known that not eating enough could lead to malnutrition and this can lead to low blood sugar and vitamin deficiency. As a consequence to this, the body can become shaky. Similarly, dehydration can cause uncontrolled shaking. This is especially evident in the hands of a subject.

It is therefore possible to monitor shaking (i.e. micro movements) as an indicator of physiological wellbeing, and in particular of malnutrition or dehydration, but there are other possible causes which need to be distinguished, such as emotions and responses caused by the activity being undertaken. The invention makes of additional monitoring, in particular of biological (i.e. physiological) parameters such as galvanic skin response and heart rate, and also of performance information in respect of the activity being undertaken such as a measure of the rate of making mistakes in the activity. By processing these three specific types of information, it can be accurately and reliably determined whether a physiological wellbeing of a user has deteriorated. If a user's physiological wellbeing has deteriorated and yet the user is continuing to perform the activity, this will typically indicate that a user has become dehydrated or malnourished, having become so absorbed in the activity that they have forgotten to take a break to eat and/or drink.

In some examples, the processing comprises comparing the performance information to historical performance information.

In some examples, the processing comprises comparing the shakiness information to historical shakiness information.

This historical performance information and historical shakiness information could be a user's average, a population average, a trailing average, a clinically informed threshold, etc.

In some examples, the historical information is information in respect of a time with the user hydrated and well-fed.

This allows the historical information to be user-specific, allowing a more reliable assessment of the physiological wellbeing of a specific user.

In some examples, the processing comprises determining a correlation between the shakiness information and the biological information.

This allows shakiness due to factors other than dehydration or malnourishment, e.g., an intense section of the activity, to be identified and subsequently discounted in determining a physiological wellbeing value.

In some examples, the biological parameters comprise at least one of heart rate and galvanic skin response.

These parameters are able to be measured by dry electrodes which are common in smartwatches and wearable devices. They are also able to inform on a user's stress response which may be used to assess whether a user's shakiness is due to factors other than a deterioration in physiological wellbeing.

In some examples, the activity is a game, and the performance information includes performance metrics in the game.

Users often forget to eat and drink when playing games, and so the invention may be particularly beneficial to users while they are playing games. Users making mistakes while playing their games, which may easily be monitored via performance metrics in the game such as a kill to death ratio, progress per time-interval, etc., may indicate a deterioration in a user's physiological wellbeing.

In some examples, the method further comprises: generating at least one recommendation to the user based on the determined physiological wellbeing value; and presenting the generated at least one recommendation to the user.

Once a physiological wellbeing value has been determined, a recommendation to the user can be generated and presented to the user to prompt them into taking action that may aid them.

In some examples, the at least one recommendation comprises at least one of drinking and eating.

A deterioration in a user's physiological wellbeing during an activity is typically a result of dehydration and/or malnutrition, due to forgetting to eat or drink while performing the activity. If a determined physiological wellbeing value indicates a deterioration in wellbeing and the user continues to perform the activity, it may be inferred that the user is dehydrated and/or malnourished, since it is unlikely that the user would continue to perform the activity if a more severe medical issue (e.g. a heart attack or a stroke) had caused the deterioration in physiological wellbeing. A recommendation that the user eat or drink may thus be generated and presented to the user to prompt them to remedy their dehydration and/or malnutrition.

In some examples, the method further comprises generating a control signal to control the system which the user operates to perform the activity based on the generated recommendation.

In order to ensure the user performs the presented recommendation, a control signal may be generated to control the system which is operating the user's activity, for instance, to pause a game until it is determined that the user's physiological wellbeing has improved.

There is also proposed a computer program comprising code means for implementing the method described above when said program is run on a processing system.

According to another aspect of the invention, there is provided a system for tracking physiological wellbeing of a user during an activity, the system comprising: an input interface configured to receive shakiness information, biological information, and performance information; and a processor arrangement configured to: process the shakiness information, biological information, and performance information to determine a physiological wellbeing value.

In some examples, the system further comprises at least one movement sensor for generating the shakiness information, wherein the at least one movement sensor comprises at least one of a touch sensor, an Inertial Measurement Unit, and an accelerometer.

These sensors allow micro-movements to be measured and are also common in devices such as game controllers, smartphones, or smartwatches, which may already be used in the user's activity.

In some examples, the at least one movement sensor comprises part of a smartphone, a smartwatch, a game controller, or a wearable device.

Wearable devices that many users already wear commonly include biological sensors capable of measuring heart rate and/or galvanic skin response.

In some examples, the system further comprises at least one biological sensor for generating the biological information, wherein the at least one biological sensor comprises part of a wearable device.

Wearable devices that many users already wear commonly include biological sensors capable of measuring heart rate and/or galvanic skin response.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates a system for tracking physiological wellbeing of a user during an activity, according to the embodiment of the invention; and
Fig. 2 illustrates a computer-implemented method for tracking physiological wellbeing of a user during an activity, according to an embodiment of the invention.

### DETAILED DESCRIPTION OF THE EMBODIMENTS

The invention will be described with reference to the Figs.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figs are merely schematic and are not drawn to scale. It should also be understood that the same reference numerals are used throughout the Figs to indicate the same or similar parts.

The invention provides a method and system for tracking physiological wellbeing of a user during an activity. Information relating to micro-movements, biological parameters and activity performance of the user during the activity is received and processed to determine a physiological wellbeing value.

Embodiments are at least partly based on the realization that a combination of information relating to micro-movements of a user, information relating to biological parameters of the user and information relating to a level of performance of the user in performing an activity allows the physiological wellbeing of the user during the activity to be monitored more accurately and reliably. In particular, the inventors have recognized that this combination of information both identifies symptoms of dehydration or malnutrition and allows an assessment to be made as to whether these symptoms are due to other factors unrelated to physiological wellbeing.

Illustrative embodiments may, for example, be employed in personal health monitoring applications, gaming systems and other systems for which a user may forget to eat or drink while operating the system, thus adversely affecting their physiological wellbeing.

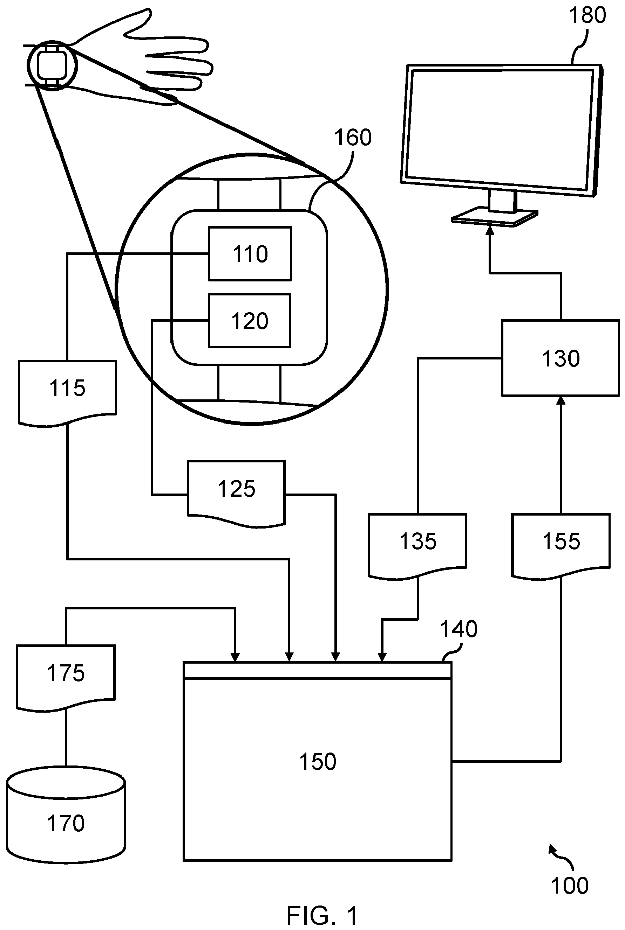

Fig. 1 illustrates a system 100 for tracking physiological wellbeing of a user during an activity, according to the embodiment of the invention. The activity may be any activity that a user spends long periods of time performing, during which the user may forget to eat or drink, and for which a level of performance of the user may be monitored. For example, the activity may be a computer game or screen work (e.g., working on a computer).

The system 100 comprises at least one movement sensor 110, at least one biological sensor 120, a system 130 which the user operates to perform the activity, an input interface 140 and a processor arrangement 150.

In Fig. 1, the at least one movement sensor 110 and the at least one biological sensor 120 each comprise part of smartwatch 160. As will be apparent to the skilled person, the at least one movement sensor and the at least one biological sensor may be contained in any suitable device, and sensors may be located in separate devices. For instance, the at least one movement sensor may comprise part of a smartphone, a game controller, a smartwatch or any other suitable wearable device, while the at least one biological sensor may comprise part of a smartwatch or any other suitable wearable device. In some examples, at least one movement and/or biological sensor may be contained in a device that forms part of the system 130 which the user operates to perform the activity (e.g. a game controller, where the activity is a game).

The at least one movement sensor 110 generates shakiness information 115 relating to micro-movements of the user during the activity. Micro-movements (or "micro-motions") are tremors (uncontrolled shaking) that occur when muscle power is needed but the body is unable to maintain recruitment of muscle fibers at a required rate (e.g. when holding the body in a position requiring muscle contraction for an extended time). These micro-movements can be caused by dehydration or malnutrition, as the muscles are not receiving the energy required to perform the activity.

The at least one movement sensor 110 may comprise at least one of a touch sensor, an Inertial Measurement Unit (IMU) and/or an accelerometer. The shakiness information may therefore comprise touch data from a touch sensor and/or motion data from an IMU and/or accelerometer. IMUs and accelerometers are commonly used to detect motion, and are already present in many devices that users wear (e.g. smartwatches) and in many devices that may be used in the user's activity (e.g. game controllers and smartphones). Further suitable sensors for obtaining information relating to micro-movements of a user will be readily apparent to the skilled person.

The at least one biological sensor 120 generates biological information 125 relating to biological parameters of the user during the activity. The biological parameters are physiological parameters that may be used to distinguish shakiness caused by dehydration and/or malnutrition from shakiness from other causes not related to physiological wellbeing (such as an emotional response to the activity). For example, the at least one biological sensor may comprise a dry electrode sensor, and the biological parameters may comprise at least one of heart rate and/or galvanic skin response. Heart rate increases with dehydration to compensate for fluid loss (as amount of blood circulating decreases), as does galvanic skin response (as less fluid means higher impedance).

Dry electrodes are commonly found in wearable devices such as smartwatches, so the system 100 can make use of a device already worn by the user to obtain the biological information. Further suitable sensors for obtaining biological information relating to biological parameters will be readily apparent to the skilled person.

The system 130 which the user operates to perform the activity generates performance information 135 relating to a level of performance of the user in performing the activity. The type of performance information generated by the system 130 may depend on the type of activity being performed. For instance, if the activity is a game, the performance information may include performance metrics in the game, such as a kill-to-death ratio and/or a progress per time interval, and/or measures of interacting with the game (e.g. frequency of button presses). If the activity involves typing, the performance information may include a typing speed and/or a measure of mistakes made by the user (e.g. a frequency of typos, mistake corrections and/or typed words that are not recognized by a spellchecker).

The input interface 140 receives the shakiness information 115 from the at least one movement sensor 110, the biological information 125 from the at least one biological sensor 120 and the performance information 135 from the system 130 which the user operates to perform the activity. The processor arrangement 150 processes the shakiness information, biological information and performance information to determine a physiological wellbeing value. A physiological wellbeing value is a value representative of a level of physiological wellbeing of the user, and may be indicative of a level of dehydration and/or malnutrition of the user.

The processor arrangement 150 may process the shakiness information 115 obtained by the at least one movement sensor 110 to detect micro-movements in the shakiness information. The processor arrangement 150 may then process the detected micro-movements to determine at least one measure of shakiness for the user, such as a frequency of micro-movements, or a moving average of an amplitude of micro-movements (e.g. an average over the previous 10 seconds, 1 minute, etc.).

The use of IMUs and accelerometers to measure tremors in patients with essential tremor and Parkinson's disease is known (see, for example, Vescio, B. et al. (2021), "Wearable devices for assessment of tremor", Frontiers in Neurology, 12:680011; Fuchs, C. et al. (2021), "Tremor assessment using smartphone sensor data and fuzzy reasoning", BMC Bioinformatics, 22(Suppl 2):57; and van Brummelen, E. M. J. (2020), "Quantification of tremor using consumer product accelerometry is feasible in patients with essential tremor and Parkinson's disease: a comparative study", J Clin Mov Disord, 7:4); the skilled person will therefore understand how to detect micro-movements in movement data from an IMU and/or accelerometer.

Micro-movements may additionally or alternatively be detected by detecting oscillations in data from a touch sensor (e.g. oscillations between touching and not touching, or oscillations in the position at which a touch is detected, if the touch sensor has a sufficiently high resolution), and measuring the pressure exerted when pressing analog buttons.

The processor arrangement 150 may process the biological information 125 obtained by the at least one biological sensor 120 to determine a value for each biological parameter. For example, the processor arrangement may process data obtained by a dry electrode sensor to determine a value for at least one of heart rate and/or galvanic skin response.

The use of biological sensors to measure biological parameters is well-known, and how to determine the value of a biological parameter from data obtained by a biological sensor will be readily apparent to the skilled person.

The processor arrangement 150 may process the performance information 135 obtained by the system 130 which the user operates to perform the activity to determine at least one measure of performance for the user. Alternatively, at least one measure of performance for the user may be determined by the system 130, and the performance information may comprise the at least one measure of performance for the user (e.g. performance metrics in a game, typing speed and/or a measure of mistakes made by the user for a typing activity). When the activity is a game, the at least one measure of performance may be obtained by linking the user's gaming account to the processor arrangement 150 (e.g. the user's Steam^{®} account). The user's gaming account may also register a duration of a current session of the game. This information may, for example, be used to detect the start and end of a gaming session, and thus to determine when to start and stop tracking the user's physiological wellbeing.

The processor arrangement 150 may determine a physiological wellbeing value by comparing at least one of the shakiness information 115, the biological information 125 and the performance information 135 with historical information 175. The historical information 175 may be obtained from a memory unit 170.

The historical information 175 may comprise at least one of historical shakiness information, historical biological information and/or historical performance information. The historical information may comprise one or more historical values for a measure of shakiness, biological parameters and/or a measure of performance.

For instance, the processor arrangement 150 may process the shakiness information 115 by comparing the shakiness information to historical shakiness information, e.g. by comparing a current value for one or more measures of shakiness for the user with corresponding historical value for the one or more measures of shakiness. Similarly, the processor arrangement may process the biological information 125 by comparing the biological information to historical biological information (e.g. by comparing a current value for each biological parameter for the user with a corresponding historical value for each biological parameter), and process the performance information 135 by comparing the performance information to historical performance information (e.g. by comparing a value for one or more measures of performance for the user with corresponding historical value for the one or more measures of performance).

For example, the comparison with historical information may be used to determine whether there has been a decrease in a user's performance metric (e.g. average score) and/or an increase in the user's micromovements, galvanic skin response, average heart rate, fluctuations in input (when using analog buttons) and/or errors. The physiological wellbeing value may be a binary value according to whether any or all of these trends (or a subset of these trends) are detected, or a decimal value/percentage indicating a likelihood of a deterioration in the user's physiological wellbeing according to how many of these trends are detected. For instance, a physiological wellbeing value indicating a high likelihood of a deterioration in wellbeing may be generated in response to determining that a user's performance metric has decreased and their micromovements, galvanic skin response and average heart rate have increased (e.g. by more than a predetermined percentage). A physiological wellbeing value indicating a low likelihood of a deterioration in wellbeing may be generated in response to determining that the user is exhibiting an increase in micromovements but that there is no change (or no significant change) in the user's biological parameters and level of performance.

A historical value for a measure of shakiness, biological parameter or measure of performance may, for example, be a user average, a population average, a trailing average or a clinically informed threshold. Other suitable historical values will be apparent to the skilled person.

In some examples, the historical information is user-specific historical information (e.g. historical user averages) in respect of a time with the user hydrated and well-fed. The system 100 may, e.g. during a first use by the user, output a request to the user to eat and drink before performing the activity, and obtain shakiness information, biological information and/or performance information, as described above, while the user performs the activity having eaten and drunk. The processor arrangement 150 may receive the shakiness information, biological information and/or performance information via the input interface 140, and process the shakiness information, biological information and/or performance information to generate the historical information 175. The historical information may then be stored in the memory unit 170 for future activity sessions.

The system 100 may obtain historical information only at a time at which the user is hydrated and well-fed, or obtain historical information throughout a session of the activity that began with the user being hydrated and well-fed. Alternatively, the system 100 may request a user input (e.g. via a user-input device forming part of the system 130 which the user operates to perform the activity) providing a time since the user last ate and/or drank. A request to the user to eat and drink, or to provide a user input, may be communicated to the user via an output user interface connected to or forming part of the system 130 which the user operates to perform the activity, such as the display device 180 in Fig. 1.

In some examples, the determination of a physiological wellbeing value may additionally or alternatively comprise processing the shakiness information 115 and the biological information 125 to determine a correlation between the shakiness information and the biological information, in order to account for micro-movements caused by factors other than a deterioration in physiological wellbeing, such as an increase in activity intensity and/or an emotional response to the activity.

In some examples, the shakiness information 115 and biological information 125 may be processed to determine a correlation in response to a comparison with historical shakiness information indicating that the user's physiological wellbeing may have deteriorated. In other words, the processor arrangement may first compare the received shakiness information with historical information, and determine a correlation between the shakiness information and the biological information only if the comparison between the shakiness information and the historical information indicates that the user's shakiness has increased (e.g. to above a predetermined shakiness threshold or by more than a predetermined percentage).

In some examples, the processor arrangement 150 may use a machine-learning algorithm to determine a physiological wellbeing value based on the shakiness information 115, biological information 125 and performance information 135.

A machine-learning algorithm is any self-training algorithm that processes input data in order to produce or predict output data. Here, the input data may comprise the shakiness information 115, biological information 125 and performance information 135 (or values of measures/parameters derived from this information) and the output data may comprise a physiological wellbeing value.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

The training input data entries may correspond to example shakiness information, biological information and performance information (or values of measures/parameters derived from this information). The training output data entries may correspond to physiological wellbeing values. The training data may be population data and/or user-specific data. The use of user-specific data allows a machine-learning algorithm to learn how micro-movements, biological parameters and performance of the user changes over time for that user as physiological wellbeing of the user deteriorates as the user becomes dehydrated/malnourished.

The use of a machine-learning algorithm may allow dehydration and/or malnutrition to be detected at an early stage, so that action can be taken before the dehydration and/or malnutrition becomes too severe. This is both beneficial for the user's health and allows the user to maintain a high performance (which may be particularly valuable to users in the case of gaming, where the user does not want their performance statistics to deteriorate).

Having determined the physiological wellbeing value, the processor arrangement 150 may process the determined physiological wellbeing value to generate at least one recommendation to the user. The recommendation may comprise at least one of drinking and eating. In particular, the recommendation may comprise at least one of drinking and eating in response to the determined physiological wellbeing value indicating that the user's physiological wellbeing has deteriorated, e.g. in response to the determined physiological wellbeing value breaching a predetermined threshold. A deterioration in a user's physiological wellbeing during an activity is most likely due to dehydration and/or malnutrition, as a user would most likely stop performing the activity if they experienced a more severe medical issue having similar symptoms to dehydration/malnutrition (such as a heart attack or stroke).

Other suitable recommendations will be apparent to the skilled person. For instance, the recommendation may comprise a suggestion that the user takes a break from the activity. In some examples, the recommendation may include a reason for the recommendation (e.g. that the user's performance of the activity has deteriorated).

The at least one recommendation may be presented to the user, for example, by providing a user-perceptible output to an output user interface connected to or forming part of the system 130 which the user operates to perform the activity, such as the display device 180 in Fig. 1. For instance, the at least one recommendation may be presented to the user in the form of a textual display, an image and/or a sound. Further examples of suitable user-perceptible outputs will be apparent to the skilled person.

In some examples, the processor arrangement 150 may process the generated recommendation to generate a control signal 155 to control the system 130 which the user operates to perform the activity. In particular, the processor arrangement may generate a control signal to control the system 130 to prevent the user performing the activity until the user's physiological wellbeing has improved (e.g. until the user is no longer dehydrated/malnourished). The system 100 may continue to obtain shakiness information and/or biological information while the user is prevented from performing the activity in order to determine whether there has been any improvement in the user's physiological wellbeing.

For instance, if the activity is a game, the processor arrangement 150 may process the generated recommendation to generate a control signal to control the system 130 to pause the game until the user is no longer determined to have poor physiological wellbeing. If the activity is deskwork, the processor arrangement may generate a control signal to lock or reduce access to the user's screen.

For improved clarity, the device containing the at least one movement sensor 110 and the at least one biological sensor 120, the system 130 which the user operates to perform the activity and the processor arrangement 150 are shown as separate elements. However, the skilled person will readily appreciate that one or more of these elements may form a single device/system. For instance, the processor arrangement 150 may form part of the system 130 which the user operates to perform the activity and/or part of a device containing the at least one movement sensor 110 and/or the at least one biological sensor 120.

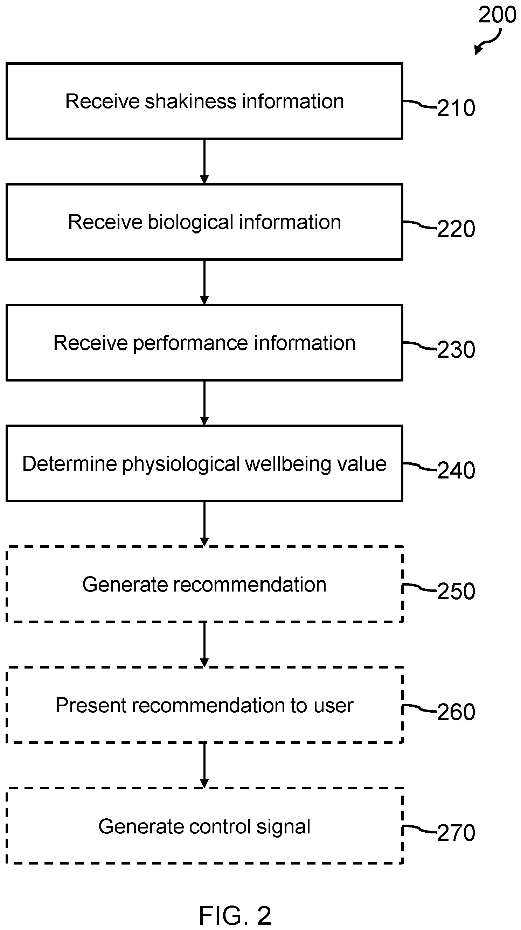

Fig. 2 illustrates a computer-implemented method 200 for tracking physiological wellbeing of a user during an activity, according to an embodiment of the invention.

The method begins with step 210, at which shakiness information relating to micro-movements of the user is received from at least one movement sensor.

At step 220, biological information relating to biological parameters of the user is received from at least one biological sensor. The biological parameters may comprise at least one of heart rate and/or galvanic skin response.

At step 230, performance information relating to a level of performance of the user in performing the activity is received from a system which the user operates to perform the activity. In some examples, the activity is a game, and the performance information includes performance metrics in the game.

At step 240, the shakiness information, biological information and performance information are processed to determine a physiological wellbeing value. The processing may comprise comparing the performance information to historical performance information and/or comparing the shakiness information to historical shakiness information. The historical information may be information in respect of a time with the user hydrated and well-fed. In some examples, the processing may comprise determining a correlation between the shakiness information and the biological information.

In some examples, the method 200 may further comprise a step 250, at which at least one recommendation to the user is generated based on the determined physiological wellbeing value, and a step 260, at which the generated at least one recommendation is presented to the user. The at least one recommendation may comprise at least one of drinking and/or eating.

In some examples, the method 200 may further comprise a step 270, at which a control signal to control the system which the user operates to perform the activity is generated based on the generated recommendation.

It will be understood that the disclosed methods are computer-implemented methods. As such, there is also proposed a concept of a computer program comprising code means for implementing any described method when said program is run on a processing system.

The skilled person would be readily capable of developing a processor for carrying out any herein described method. Thus, each step of a flow chart may represent a different action performed by a processor, and may be performed by a respective module of the processor.

As discussed above, the system makes use of a processor to perform the data processing. The processor can be implemented in numerous ways, with software and/or hardware, to perform the various functions required. The processor typically employs one or more microprocessors that may be programmed using software (e.g. microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.
Any reference signs in the claims should not be construed as limiting the scope.

## Claims

1. A method for tracking physiological wellbeing of a user during an activity, the method comprising:
receiving shakiness information relating to micro-movements of the user from at least one movement sensor;
receiving biological information relating to biological parameters of the user from at least one biological sensor;
receiving performance information relating to a level of performance of the user in performing the activity from a system which the user operates to perform the activity; and
processing the shakiness information, biological information, and performance information to determine a physiological wellbeing value.

2. The method of claim 1, wherein the processing comprises comparing the performance information to historical performance information.

3. The method of claim 1 or 2, wherein the processing comprises comparing the shakiness information to historical shakiness information.

4. The method of claim 2 or 3, wherein the historical information is information in respect of a time with the user hydrated and well-fed.

5. The method of any one of claims 1 to 4, wherein the processing comprises determining a correlation between the shakiness information and the biological information.

6. The method of any one of claims 1 to 5, wherein the biological parameters comprise at least one of heart rate and galvanic skin response.

7. The method of any one of claims 1 to 6, wherein the activity is a game, and the performance information includes performance metrics in the game.

8. The method of any one of claims 1 to 7, wherein the method further comprises:
generating at least one recommendation to the user based on the determined physiological wellbeing value; and
presenting the generated at least one recommendation to the user.

9. The method of claim 8, wherein the at least one recommendation comprises at least one of drinking and eating.

10. The method of claim 8 or 9, further comprising:
generating a control signal to control the system which the user operates to perform the activity based on the generated recommendation.

11. A computer program comprising code means for implementing the method of any one of claims 1 to 10 when said program is run on a processing system.

12. A system for tracking physiological wellbeing of a user during an activity, the system comprising:
an input interface configured to receive shakiness information, biological information, and performance information; and
a processor arrangement configured to:
process the shakiness information, biological information, and performance information to determine a physiological wellbeing value.

13. The system of claim 12, further comprising at least one movement sensor for generating the shakiness information, wherein the at least one movement sensor comprises at least one of a touch sensor, an Inertial Measurement Unit, and an accelerometer.

14. The system of claim 13, wherein the at least one movement sensor comprises part of a smartphone, a smartwatch, a game controller, or a wearable device.

15. The system of any one of claims 12 to 14, further comprising at least one biological sensor for generating the biological information, wherein the at least one biological sensor comprises part of a wearable device.
